# EUROPEAN PATENT APPLICATION

(11) **EP 3 366 764 A1**
(43) Date of publication of application: **29.08.2018**
(21) Application number: 17157637.4
(22) Date of filing: 23.02.2017
(51) Int. Cl.: C12M 1/107, C12M 1/34, C12M 1/36, C12P 5/02

(54) **ANAEROBIC DIGESTION SYSTEM FOR PRODUCTION OF BIOGAS WITH A REDUCED HYDROGEN SULPHIDE CONTENT AND METHOD FOR PRODUCTION OF BIOGAS WITH A REDUCED HYDROGEN SULPHIDE CONTENT IN AN ANAEROBIC DIGESTION SYSTEM**

(71) Applicant: Yara International ASA, 0277 Oslo (NO)
(72) Inventor: FRANKE, Wolfram, 3917 Porsgrunn (NO); DOPPELBAUER, Günter, 47445 Moers (DE)
(74) Representative: Pappaert, Kris

(57) **Abstract**

The application relates to an anaerobic digestion system for production of biogas with a reduced hydrogen sulphide content, comprising an airtight reactor vessel comprising a sludge comprising a surface layer; and a volume underneath the surface layer comprising bio-matter and micro-organisms that are able to anaerobically digest the bio-matter producing biogas, the biogas comprising hydrogen sulphide, a headspace above the surface layer of the sludge into which biogas migrates out of the sludge, a spraying system for spraying an aqueous nitrate containing solution in the headspace using a carrier gas stream to form a nitrate containing interphase layer between the surface layer of the sludge and the headspace, wherein the interphase layer comprises the same species of micro-organisms as the ones present in the sludge, and wherein at least part of the nitrate of the interphase layer is used by the micro-organisms present in the interphase layer to oxidize at least part of the hydrogen sulphide present in the produced biogas to sulphate or sulphur, resulting in the headspace being filled with biogas with a reduced hydrogen sulphide content, with the proviso that the reactor vessel does not comprise a membrane or fabric positioned in the headspace. The application also relates to a method for production of biogas with a reduced hydrogen sulphide content in an anaerobic digestion system.

## Description

### Technical field

The application relates to the technical field of hydrogen sulfide mitigation in anaerobic digestion processes (also called fermenter processes) for biogas production. Anaerobic digestion is a collection of processes by which microorganisms break down biodegradable material in the absence of oxygen.

### Background

Biogas is a mixture of gases produced from the anaerobic oxidation (or digestion) of organic matter. The main components of biogas are methane (CH₄) and carbon dioxide (CO₂). During anaerobic digestion, sludge is digested (fermented) in a fermenter under anaerobic conditions. Fermenters typically contain a fermentation sludge fraction and a headspace above the fermentation sludge fraction filled with biogas.

Biogas, as a renewable energy, can be produced from a variety of organic raw materials and utilized for various energy services, such as heat, combined heat and power or as a vehicle fuel. Biogas can be produced by anaerobic digestion or fermentation of biodegradable materials such as biomass, manure, sewage, municipal waste, green waste, plants material and energy crops. Emphasis is laid on sludge from municipal wastewater treatment plants in this paper. Large amounts of waste activated sludge, containing organic and mineral components, are produced by municipal and industrial wastewater treatment plants. Most relevant is municipal solid waste, with a daily production in Europe of about 400,000 tons. Sludge handling represents a bottleneck in wastewater treatment plants, due to environmental, economic, social and legal factors. If handled properly, sludge can be a valuable resource for renewable energy production and a source of nutrients for agriculture.

Biogas is produced with the purpose of energetic use, having as a consequence that the gas quality needs to meet the technical requirements of combustion engines. Impurities such as H₂S are found in concentrations between 1.000 to 10.000 ppm, while combustion systems require typically concentrations lower than 200 ppm of H₂S. The most extended technologies to H₂S removal from biogas are downstream processes, often physic-chemical processes as wet air oxidation. These processes however have high investment and operational costs.

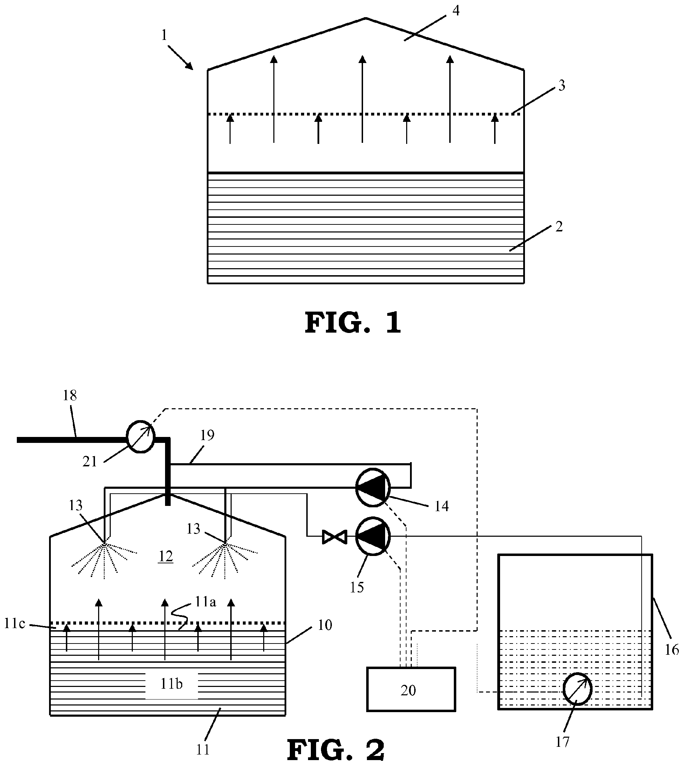

The following two options are also known as another possibility to reduce H₂S contamination in situ (on-site):
1. A common way is the precipitation of the present hydrogen sulfide in the fermenter, what is done by iron salt addition. Iron salts are usually low pH solutions and therefore very corrosive as well as difficult to handle. Alternatively, it was shown that micro dosage of oxygen or nitrate can prevent hydrogen sulphide production to some extent. This technology is however limited as too high levels of an electron donator would change the redox-potential and the fermentation process would get disturbed.
2. Another common type of in situ H₂S removal is the use of a fabric (3) installed in the headspace (4) of a fermenter (1), comprising sludge (2), as shown in FIG. 1. Microorganisms settle on the fabric (3) and take care of the oxidation of H₂S to sulphate or sulphur using added oxygen. The formed sulphur needs to be removed from the fabric (3) later on. This is usually done once a year. This system has as a disadvantage that the replacement / maintenance of the fabric (3) demands downtime of fermenter (1). Furthermore, the fermenter (1) needs to be opened which is accompanied with methane release to atmosphere. There is also no way of controlling the quality of the biogas, since the removal efficiency of this type of system depends on the colonization of the fabric (3) by the microorganisms.

It is a purpose of the application to provide in hydrogen sulfide mitigation in a simple and effective anaerobic digestion biogas production system and process without the problems of the currently known systems and processes as disclosed above.

### Summary of the invention

According to an aspect of the present application, an anaerobic digestion system for production of biogas with a reduced hydrogen sulphide content is described, comprising an airtight reactor vessel comprising
- a sludge comprising
   - a surface layer; and
   - a volume underneath the surface layer comprising organic matter and micro-organisms that are able to anaerobically digest the organic matter producing biogas comprising hydrogen sulphide;
- a headspace above the surface layer of the sludge into which purified biogas is migrating out of the sludge;
- a spraying system for spraying an aqueous nitrate containing solution in the headspace using a carrier gas stream to form a nitrate containing interphase layer between the surface layer of the sludge and the headspace, wherein the interphase layer comprises the same species of micro-organisms as the ones present in the sludge, and wherein at least part of the nitrate of the interphase layer is used by the micro-organisms present in the interphase layer to oxidize at least part of the hydrogen sulphide present in the produced biogas to sulphate or sulphur, resulting in the headspace being filled with biogas with a reduced hydrogen sulphide content;
with the proviso that the reactor vessel does not comprise a membrane or fabric positioned in the headspace.

It is important that the concentration of nitrate in the aqueous nitrate containing solution is high enough to stimulate the microbial oxidation of hydrogen sulfide to sulfate or sulfur. As used herein, the terms "reduced hydrogen sulfide content" or "practically free from hydrogen sulphide" refers to a reduction of the hydrogen sulphide content of the biogas to a hydrogen sulphide content of less than about 400 or 300 ppm, preferably less than about 200 ppm, such as less than 150 ppm or 100 ppm. In this context, combustion systems typically require concentrations lower than 200 ppm of H₂S.

In view of the anaerobic digestion systems according to the prior art, the anaerobic digestion system according to the application has the following advantages:
- Since the removal of hydrogen sulphide is performed at the surface layer of the sludge, there is a reduction of maintenance work and a reduction of the methane emissions to the atmosphere, and
- since injection or air is avoided, and consequently dilution by nitrogen is minimized, there is an increased quality of the biogas.

In an embodiment of anaerobic digestion system according to the application, the gas in the carrier gas stream is recirculated biogas. Using recycled biogas in the carrier gas stream takes care that the biogas quality is not influenced.

More specifically, the anaerobic digestion system comprises a gas tight vacuum pump to suck biogas out of the headspace of the reactor vessel or to obtain the biogas that is recycled back into the headspace of the reactor vessel.

In a possible embodiment of an anaerobic digestion system according to the application, the gas in the carrier gas stream is compressed gas from a source outside the reactor vessel, more specifically compressed nitrogen gas.

In an embodiment of an anaerobic digestion system according to the application, the spraying system comprises one or more spraying nozzles arranged to produce aqueous nitrate containing solution droplets using the carrier gas stream.

In a possible embodiment of an anaerobic digestion system according to the application, the anaerobic digestion system comprises a pipeline leading the purified biogas out of the headspace, and the anaerobic digestion system comprises a gas tight vacuum pump to suck biogas out of the headspace of the reactor vessel or the pipeline.

The one or more spraying nozzles are more in particular venturi nozzles. Venturi nozzles have the best atomizing effect for highly viscous liquids.

In case that venturi nozzles are applied, the anaerobic digestion system can possibly comprise a low speed and low pressure dosage pump to apply the aqueous nitrate containing solution to the one or more spraying nozzles. It is possible to use such a low speed and low pressure dosage pump since the atomization is done by the venturi-effect of the venture spraying nozzles.

In a possible embodiment of an anaerobic digestion system according to the application, the aqueous nitrate containing solution includes an aqueous sodium nitrate solution, an aqueous potassium nitrate solution, an aqueous calcium nitrate solution, an aqueous magnesium nitrate solution or an aqueous nitric acid solution.

An aqueous nitric acid solution (HNO₃) provides nitrate (NO₃) and hydrogen (H) and therefore, sulphuric acid (H₂SO₄) is formed when being in contact with hydrogen sulphide through a redox reaction.

If an aqueous sodium nitrate solution is used, the reaction product is the Glauber's Salt (decahydrate of Na₂SO₄) that is a crystal which is good water soluble and will stay in solution.

In particular, calcium nitrate solution is chosen since the reaction product is a poorly water soluble salt, i.e. gypsum or CaSO₄. The advantage of gypsum is that it is neutral and secondly, that it exits the reactor vessel together with the digestate.

In a possible embodiment of an anaerobic digestion system according to the application, the aqueous nitrate containing solution has a concentration of between 10 to 30 weight% of nitrate. These solutions do not clog the spraying nozzles.

According to another aspect of the application, a method is disclosed for producing biogas with a reduced hydrogen sulphide content in an anaerobic digestion system, comprising the steps of:
- providing an airtight reactor vessel;
- providing a sludge in the airtight reactor vessel having a surface layer and a volume underneath the surface layer comprising bio-matter and micro-organisms;
- anaerobically digesting the bio-matter producing biogas comprising hydrogen sulphide;
- spraying an aqueous nitrate containing solution in a headspace of the reactor vessel above the surface layer using a carrier gas stream;
- forming a nitrate containing interphase layer between the surface layer of the sludge and the headspace, the interphase layer comprising the same species of micro-organisms as the ones present in the sludge;
- purifying the biogas by oxidizing hydrogen sulphide present in the biogas to sulphate or sulphur by the micro-organisms present in the interphase layer using nitrate of the interphase layer, where after the purified biogas is migrating to the headspace;
with the proviso that no membrane or fabric is positioned in the headspace of the reactor vessel.

In a possible method according to the application, the method comprises the step of continuously measuring the residual hydrogen sulphide concentration in the purified biogas.

In possible embodiments of the method according to the application, a storage tank is provided for storing the aqueous nitrate containing solution, and wherein the method includes the step of continuously measuring the temperature of aqueous nitrate containing solution in the storage tank.

The storage tank more in particular comprises a heat source to heat the aqueous nitrate containing solution in the storage tank, wherein the method comprises the step of heating the aqueous nitrate containing solution in the storage tank if the temperature of the aqueous nitrate containing solution in the storage tank is below a predetermined temperature, in particular below 5°C.

In possible embodiments the method further possibly comprises the step of continuously measuring the level of the aqueous nitrate containing solution in the storage tank.

In possible embodiments of the method according to the application, the anaerobic digestion system comprises a low speed and low pressure dosage pump to apply the aqueous nitrate containing solution to the one or more spraying nozzles and wherein the method comprises the step of continuously controlling the start and stop of the dosage pump. This allows to stop the dosage pump to prevent overdose or to stop dosage in case the level of the aqueous nitrate containing solution in the storage tank reaches its lowest level.

In a possible embodiment of a method according to the application, the anaerobic digestion system comprises a gas tight vacuum pump to suck biogas out of the headspace of the reactor vessel, and wherein the method comprises the step of controlling the start and stop of the vacuum pump.

In an embodiment of a method according to the application, the method comprises the step of continuously measuring the dosage of the aqueous nitrate containing solution in the headspace.

According to another aspect of the application, a computer program product is disclosed comprising program code instructions for implementing a method according to the application as described above.

According to another aspect of the application, a system according to the application as described above is disclosed that is configured for executing a method according to the application as described above.

### Description of the figures

FIG. 1 shows a schematic representation of an anaerobic digestion system for biogas production according to the prior art using a fabric installed in the headspace of the reactor vessel;
FIG. 2 shows a schematic representation of an anaerobic digestion system for biogas production according to the application.

### Detailed description of the invention

The anaerobic digestion system according to the present application, as shown in FIG. 2, comprises an airtight covered reactor vessel (10). On the one hand, the reactor vessel (10) comprises an anaerobic (fermentation) sludge (also called effluent) (11) having a surface layer (11a) and a volume underneath the surface layer (11a) comprising organic matter and micro-organisms that are able to anaerobically digest the organic matter producing biogas. The biogas consists primarily of methane, carbon dioxide, and (undesired) hydrogen sulphide. On the other hand, the reactor vessel (10) comprises a headspace (12) which is filled with biogas that migrates out of the fermenting sludge (11) and rises up into the headspace (12). The reactor vessel (10) does not comprise a membrane or fabric positioned in the headspace (12).

In order to reduce the hydrogen sulphide content in the biogas rising up into the headspace (12) of the reactor vessel (10), a spraying system is installed in the headspace (12) that is arranged to spray an aqueous nitrate containing solution in the headspace (12) using a carrier gas stream. The spraying system may comprise one or more spraying nozzles (13) using the carrier gas stream to spray the aqueous nitrate containing solution in the headspace (12). The combination of the spraying nozzles (13) and the carrier gas stream takes care that the aqueous nitrate containing solution that is injected as droplets are ripped apart by the carrier gas stream and sprayed out into the headspace (12). From there, they are evenly distributed in the headspace (12) forming an aerosol or mist of aqueous nitrate containing solution in the headspace (12) that finally settles on the surface layer (11a) of the sludge (11). In this way, an interphase layer (11c) of aqueous nitrate containing solution is formed on the surface layer (11a) of the sludge (11). This interphase layer (11c) is thus situated between the surface layer (11b) of the sludge (11) and the headspace (12). The average thickness of the interphase layer (11c) as a result of aerosol precipitation ranges from about 0.01 mm to about 1 mm, preferably from about 0.05 mm to about 0.5 mm, more preferably is about 0.02 mm. More in particular, venturi nozzles (13) are applied as spraying nozzles since these have the best atomizing effect for highly viscous liquids including aqueous nitrate containing solutions.

The interphase layer (11c) is inhabited by the same species of micro-organisms as the anaerobic sludge (11). However, due to the lower redox potential in the interphase layer (11c) than in the sludge (11), the metabolism of the microorganisms in the sludge (11) is different from the metabolism of the microorganisms in the interphase layer (11c). This higher redox potential in the sludge (11) leads to hydrogen sulphide production. The biogas with the hydrogen sulphide passes through the interphase layer (11c), in which the hydrogen sulphide is oxidized to sulphates by the microorganisms present in the interphase layer (11c) using nitrate present therein. The biogas that enters the headspace (12) consequently has a reduced hydrogen sulphide content. Accordingly, hydrogen sulphide is effectively removed from the produced biogas even before it reaches the headspace (12).

The nitrate concentration in the interphase layer (11c) must be high enough to stimulate the sulphide oxidation by the present micro-organisms. The aqueous nitrate containing solution typically has a concentration of between 10 to 30 weight% of nitrate. The aqueous nitrate containing solution may include an aqueous sodium nitrate solution, an aqueous potassium nitrate solution, an aqueous calcium nitrate solution, an aqueous magnesium nitrate solution or an aqueous nitric acid solution.

More in particular, an aqueous calcium nitrate solution is sprayed into the headspace (12), through which a calcium nitrate rich interphase layer is formed between the surface layer (11a) of the sludge (11) and the headspace (12). The hydrogen sulphide that is formed and that passes through the interphase layer (11c), will be oxidized to sulphates that react with calcium, and consequently precipitate as calcium sulphate (gypsum). The precipitated gypsum is removed from the reactor vessel (10) together with the digested solid sludge fraction. This digested solid sludge fraction is more in particular continuously pumped out of the reactor vessel (10).

Since the carrier gas stream may not influence the biogas quality, the carrier gas stream more in particular can be recirculated biogas (21). Therefore, a gas tight vacuum pump (14) may be provided to suck the biogas out of the headspace (12) or a pipeline (18) leading the purified biogas out of the headspace (12), and press it through the one or more venturi nozzles (13). The carrier gas can also be a compressed gas from a source outside the reactor vessel (10) such as compressed nitrogen gas.

The aqueous nitrate containing solution is applied to the one or more venturi nozzles (13) by means of a dosage pump (15), more in particular a low pressure and low speed dosage pump. It is possible to use such a pump since the atomization of the aqueous nitrate containing solution is done via the venturi-effect of the venturi-nozzles (13) and does not need to be done by means of the pump itself. The dosage pump (15) sucks aqueous nitrate containing solution out of a covered storage tank (16).

The biogas that migrates out of the interphase layer (11c) into the headspace (12) and that is practically free from hydrogen sulphide then moves out of the headspace (12) to the pipeline (18) to either be used further as energy source or to move to a recirculation pipeline (19) to use the recirculated biogas as carrier gas for the spraying of the aqueous nitrate containing solution.

The dosage pump (15) can be controlled to prevent over dosage and to stop dosage whenever the storage tank (16) reaches a lowest level. It is important that the dosage lines are always filled with aqueous nitrate containing solution as maintenance inside the reactor vessel (10) is difficult. In addition, there is a risk that biogas could be released through empty dosage lines. The control of the dosage pump (15) can be performed by means of a control box (20). The level of the storage tank (16) can be measured using a level sensor (17) provided in the storage tank (16). If the level sensor (17) detects a lowest level in the storage tank (16), the level sensor (17) will send a signal to the control box (20) that in its turn will send a signal to the dosage pump (15) to stop dosage of aqueous nitrate containing solution to the nozzles (13).

The storage tank (16) can be arranged with a temperature sensor (not shown on the figure) to measure the temperature of the aqueous nitrate containing solution. This is especially important when the storage tank (16) is located outside. An aqueous calcium nitrate solution with 25 weight% of calcium nitrate for instance has a freezing point of about -8°C. The measurement of the temperature in the aqueous nitrate containing solution is more in particular done continuously. The storage tank (16) can furthermore be provided with a heat source (not shown on the figure) to heat the storage tank (16) when the temperature of the aqueous nitrate containing solution is below a predetermined temperature, for instance 5°C. The heat source can for instance be a heater or a loop from re-cooling units of a power station. This control can be performed by a control box (20). If the temperature sensor measures a temperature of the aqueous nitrate containing solution which is less than the predetermined temperature, a signal can be send to the control box (20) that in its turn sends a signal to the heat source to start heating the aqueous nitrate containing solution in the storage tank (16).

A hydrogen sulfide gas sensor (21) can be provided to measure the concentration of hydrogen sulfide in the biogas present in the pipeline (18) leading the purified biogas out of the headspace (12). The measurement of the concentration of hydrogen sulfide in the biogas leaving the headspace (12) is used to adjust the dosage of aqueous nitrate containing solution to the nozzles (13). This adjustment can be controlled by means of a control box (20).

Also the start and stop of the dosage pump (15) and the start and stop of the gas tight vacuum pump (14) can be controlled by a control box (20).

The anaerobic digestion system can comprise one or more control boxes (20) to perform the control over the devices and signals as mentioned in the paragraphs before.

The method according to the application involves the steps of first of all providing an airtight reactor vessel (10) having a sludge (11) with a surface layer (11a) and a volume (11b) underneath the surface layer (11a) comprising bio-matter and micro-organisms. The method further comprises the step of anaerobic digestion of the bio-matter by the micro-organisms present in the sludge (11) to produce biogas. This biogas will comprise unwanted hydrogen sulphide. The method also comprises the step of spraying an aqueous nitrate containing solution in the headspace (12) above the surface layer (11a) using a carrier gas stream. This step is followed by the formation of a nitrate containing interphase layer (11c) between the surface layer (11a) of the sludge (11) and the headspace (12). This interphase layer (11c) comprises the same species of micro-organisms as the ones present in the sludge (11). A following step is the purification of the biogas formed by the fermentation sludge (11) through oxidation of hydrogen sulphide present in the biogas to sulphate or sulphur by the micro-organisms present in the interphase layer (11c) using nitrate of the interphase layer (11c). Thereafter, the purified biogas migrates into the headspace (12). No membrane or fabric is provided in the headspace (12).

The method includes the step of continuously measuring the residual hydrogen sulphide concentration in the purified biogas, this by means of the hydrogen sulphide gas sensor (21) present in the pipeline (19) transporting the biogas out of the headspace (12).

The method can further include the step of continuously measuring the temperature of the aqueous nitrate containing solution present in the storage tank (16). The method can also comprise the step of heating the aqueous nitrate containing solution in the storage tank (16) using a heat source (not shown on the figure) when the temperature of the aqueous nitrate containing solution in the storage tank is below a predetermined temperature, in particular when it is below 5°C.

The method further possibly comprises the step of continuously measuring the level of the aqueous nitrate containing solution in the storage tank (16).

The method according to the application can also comprise the step of continuously controlling the start and stop of the low speed and low pressure dosage pump to apply the aqueous nitrate containing solution to the one or more spraying nozzles, this allowing to stop the dosage pump to prevent overdose or to stop dosage in case the level of the aqueous nitrate containing solution in the storage tank reaches its lowest level.

The method also possibly comprises the step of controlling the start and stop of the vacuum pump sucking the biogas out of the headspace of the reactor vessel.

The method according to the application may also comprise the step of continuously measuring the dosage of the aqueous nitrate containing solution in the headspace (12) of the reaction vessel (10).

The method according to the application can be computer-implemented. This allows efficient execution of the method as described above. Further can be provided a computer program product comprising program code instructions for implementing a method according to the application as described above. Accordingly, system can be efficiently programmed to execute the abovementioned method according to the application.

Also a system according to the application as described above that is configured for executing a method according to the application as described above can be provided.

### Example

In a biogas plant having an annual biogas production of 750.000 m³/yr, the H₂S concentration in the biogas is in average 2.500 ppm, what equals to 3.750 mg/m³. The approximate annual production of H₂S in the anaerobic digester used to produce the biogas is about 2.800 kg/yr. To reduce the content of H₂S in the biogas produced by the (fermentation) sludge, annually, 7.500 kg/yr of calcium nitrate is used. The dosage is in average 5.1 liter per hour when applied as a 20 wt.% aqueous calcium nitrate solution. The 20 wt.% aqueous calcium nitrate solution is made from a 45 wt.% aqueous calcium nitrate solution by dilution prior to injection via a venturi nozzle spray injection system resulting in an aerosol that precipitates on the surface layer (11a) of the sludge (11). The sludge (11) in the anaerobic digester has a surface of 314 m². The average thickness of the interphase layer (IIc) as a result of aerosol precipitation is about 0.02 mm. The advantage of adding the aqueous calcium nitrate solution using the venturi nozzle spray injection system is that the addition of calcium nitrate in the headspace (12) is restricted through which, once the aerosol (or mist) settles on the surface layer (11a) of the sludge (11), the concentration of calcium nitrate in the interphase layer (11c) will also be restricted. Consequently, the calcium nitrate will be consumed by the microorganisms close to the surface layer (11a) and will not sink deeper into the sludge (11). Furthermore, despite of the fact that the sludge (11) typically is mixed, the calcium nitrate is consumed before it can be mixed along with the sludge (11) since the mixing speed is considerably low.

The H₂S-concentration in the biogas present in the headspace of the reactor vessel is reduced to the desired level of 200 ppm.

## Claims

1. Anaerobic digestion system for production of biogas with a reduced hydrogen sulphide content, comprising an airtight reactor vessel comprising
- a sludge comprising
• a surface layer; and
• a volume underneath the surface layer comprising bio-matter and micro-organisms that are able to anaerobically digest the bio-matter into biogas, the biogas comprising hydrogen sulphide;
- a headspace above the surface layer of the sludge into which purified biogas migrates out of the sludge;
- a spraying system for spraying an aqueous nitrate containing solution in the headspace using a carrier gas stream to form a nitrate containing interphase layer between the surface layer of the sludge and the headspace, wherein the interphase layer comprises the same species of micro-organisms as the ones present in the sludge, and wherein at least part of the nitrate of the interphase layer is used by the micro-organisms present in the interphase layer to oxidize at least part of the hydrogen sulphide present in the produced biogas to sulphate or sulphur, resulting in the headspace being filled with biogas with a reduced hydrogen sulphide content;
with the proviso that the reactor vessel does not comprise a membrane or fabric positioned in the headspace.

2. Anaerobic digestion system according to claim 1, wherein the gas in the carrier gas stream is recirculated biogas or compressed gas from a source outside the reactor vessel, more specifically compressed nitrogen gas.

3. Anaerobic digestion system according to claim 2, wherein the anaerobic digestion system comprises a pipeline leading the purified biogas out of the headspace, and wherein the anaerobic digestion system comprises a gas tight vacuum pump to suck biogas out of the headspace of the reactor vessel or the pipeline.

4. Anaerobic digestion system according to any one of claims 1 to 3, wherein the spraying system comprises one or more spraying nozzles, more specifically venturi nozzles, arranged to produce aqueous nitrate containing solution droplets using the carrier gas stream.

5. Anaerobic digestion system according to claim 3 or 4, wherein a gas tight vacuum pump is connected to the one or more spraying nozzles to press the recirculated biogas serving as the carrier gas stream through the one or more spraying nozzles.

6. Anaerobic digestion system according to claim 4, wherein the anaerobic digestion system comprises a low speed and low pressure dosage pump to apply the aqueous nitrate containing solution to the one or more venturi nozzles.

7. Anaerobic digestion system according to any one of claims 1 to 6, wherein the aqueous nitrate containing solution includes an aqueous sodium nitrate solution, an aqueous potassium nitrate solution, an aqueous calcium nitrate solution, an aqueous magnesium nitrate solution or an aqueous nitric acid solution and/or wherein the aqueous nitrate containing solution has a concentration of between 10 to 30 weight% of nitrate.

8. Method for producing biogas with a reduced hydrogen sulphide content in an anaerobic digestion system, comprising the steps of:
- providing an airtight reactor vessel;
- providing a sludge in the airtight reactor vessel having a surface layer and a volume underneath the surface layer comprising bio-matter and micro-organisms;
- anaerobically digesting the bio-matter producing biogas comprising hydrogen sulphide;
- spraying an aqueous nitrate containing solution in a headspace of the reactor vessel above the surface layer using a carrier gas stream;
- forming a nitrate containing interphase layer between the surface layer of the sludge and the headspace, the interphase layer comprising the same species of micro-organisms as the ones present in the sludge;
- purifying the biogas by oxidizing hydrogen sulphide present in the biogas to sulphate or sulphur by the micro-organisms present in the interphase layer using nitrate of the interphase layer, whereafter the purified biogas is migrating to the headspace;
with the proviso that no membrane or fabric is positioned in the headspace of the reactor vessel.

9. Method according to claim 8, wherein the method comprises the step of continuously measuring the residual hydrogen sulphide concentration in the purified biogas.

10. Method according to claim 8 or 9, wherein a storage tank is provided for storing the aqueous nitrate containing solution, and wherein the method includes the step of continuously measuring the temperature of the aqueous nitrate containing solution in the storage tank and/or, wherein the method comprises the step of continuously measuring the level of the aqueous nitrate containing solution in the storage tank.

11. Method according to claim 10, wherein the storage tank comprises a heat source to heat the aqueous nitrate containing solution in the storage tank, wherein the method comprises the step of heating the aqueous nitrate containing solution in the storage tank if the temperature of the aqueous nitrate containing solution in the storage tank is below a predetermined temperature, in particular below 5°C.

12. Method according to any one of claims 8 to 11, wherein the anaerobic digestion system comprises one or more of the following:
- a low speed and low pressure dosage pump to apply the aqueous nitrate containing solution to the one or more spraying nozzles and wherein the method comprises the step of continuously controlling the start and stop of the dosage pump; and/or
- a gas tight vacuum pump to suck biogas out of the headspace of the reactor vessel, and wherein the method comprises the step of controlling the start and stop of the vacuum pump.

13. Method according to any one of claims 8 to 12, wherein the method comprises the step of continuously measuring the dosage of the aqueous nitrate containing solution in the headspace of the reactor vessel.

14. Computer program product comprising program code instructions for implementing a method according to any one of claims

15. System according to any one of claims 1 to 7 configured for executing a method according to any one of claims 8 to 13.
